# EUROPEAN PATENT SPECIFICATION

(11) **EP 2 915 877 B1**
(45) Date of publication and mention of the grant of the patent: **28.09.2016**
(21) Application number: 13850180.4
(22) Date of filing: 29.10.2013
(51) Int. Cl.: C12N 1/12, C12M 1/00, C12M 1/04, C10L 1/02, C11B 1/00, C10G 1/02

(54) **PROCESS FOR PRODUCING BIOMASS AND PRODUCTS DERIVED THEREFROM BY CULTIVATING UNICELLULAR ALGAE IN AN AQUEOUS MEDIUM SUPPLIED WITH A CO2 CURRENT, AND PLANT DESIGNED FOR THIS PURPOSE**
VERFAHREN ZUR HERSTELLUNG VON BIOMASSE UND DAVON ABGELEITETEN PRODUKTEN DURCH KULTIVIERUNG EINZELLIGER ALGEN IN EINEM MIT EINEM CO2-STROM VERSORGTEN WÄSSRIGEN MEDIUM SOWIE ANLAGE FÜR DIESEN ZWECK
PROCÉDÉ DE PRODUCTION DE BIOMASSE ET DE PRODUITS DÉRIVÉS DE CELLE-CI PAR CULTURE D'ALGUES UNICELLULAIRES DANS UN MILIEU AQUEUX ALIMENTÉ PAR UN COURANT DE CO2, ET INSTALLATION CONÇUE À CET EFFET

(30) Priority: 30.10.2012 ES 201231661
(43) Date of publication of application: 09.09.2015
(60) Divisional application: 16169346.0
(73) Proprietor: Biosinkco2 Tech Lda., B-9000-019 Funchal, Madeira (PT)
(72) Inventor: ESCUDERO CAMPILLO, Pedro, E-30740 San Pedro del Pinatar (Murcia) (ES); GOMIS CATALA, Cristian Jose, E-30740 San Pedro del Pinatar (Murcia) (ES); ESTEVE SALA, Jorge Vicente, E-30740 San Pedro del Pinatar (Murcia) (ES); CARRASCO MARTINEZ, Carlos Maria, E-30740 San Pedro del Pinatar (Murcia) (ES)
(74) Representative: Ungria López, Javier
(86) International application number: PCT/ES2013/070750
(87) International publication number: WO 2014/068161

(56) References cited:
- WO-A1-2010/021753
- WO-A1-2010/021753
- WO-A2-2010/046115
- US-A- 4 341 038
- BILLER P ET AL.: 'Potential yields and properties of oil from the hydrothermal liquefaction of microalgae with different biochemical content' BIORESOURCE TECHNOLOGY vol. 102, no. 1, 01 January 2011, pages 215 - 225, XP027368479

## Description

### Field of the invention

The present invention falls within the field of modern biotechnology applied to algae, specifically Phycotechnology. More specifically, it falls within the area of developing clean technologies for purifying and capturing harmful gases or greenhouse gases, as well as in the area concerned with producing biomass from unicellular algae for the industrial manufacture of processed and energetic products for consumption. The main application focuses on the transformation of greenhouse gases, especially CO₂, into biomass by means of the (ultraintensive) cultivation of certain species of unicellular algae, including several different strains simultaneously in monoculture, in closed systems such as vertical annular photobioreactors. This makes it possible to obtain from the biomass other products that are incorporated into the food chain, mainly lipids and proteins, before obtaining final biofuel.

In this way the intentions is to achieve an energy-efficient process and prevent the emission of liquid and gas pollutants into the environment.

### State of the art

It has been found that average energy needs at the global level have snow increased from 2300 kcal/day per person in 1980 to 2800 kcal/day per person in 2010. Furthermore, this figure is estimated to reach 3100 kcal/day per person by 2030. This, combined with the population growth that is being recorded, has pushed us to search for alternatives that can complement traditional food chain systems.

In parallel, there is growing concern about the thinning of the ozone layer and to the need of reducing emissions of greenhouse gases, among which the most important are CO₂ and CH₄. Finally, the post-petroleum era is coming closer and closer, and for this reason we are working on finding energy alternatives that are effective and not largely dependent on weather conditions.

Unicellular algae do not compete with any current staple food, they have an outstanding reproductive capacity and only need sun, a few nutrients and CO₂, in addition to water, in order to be cultivated. This water should be salty, so that they do not compete with the drinking water needs of the population.

Patent application EP 2371940 A1 discloses a process and an installation for the production of biofuels and reduction of CO₂ from punctual sources of emission. To this end, it employs non-annular tubes measuring less than 0.1 m in diameter and up to 80 metres in length. This does not lead to equal irradiation conditions across the tubes, resulting in a low surface/volume ratio, which in turn causes biomass production to decrease proportionally. In addition, the treatment of the biomass obtained in the process is carried out conventionally, wherein compounds and processed products such bio-oil or biofuel are obtained jointly.

This kind of process, such as that presented in application EP 2371940 A1, requires a drying step between the steps of separation of the biomass and the extraction of compounds, which is energy inefficient.

Document US 2008086938 A1 focuses on the overall description of the CO₂ collecting process and subsequent recycling into biofuel. In this case, also after obtaining biomass, biofuel is obtained in a single process.

International patent application WO 2007147028 A2 describes the necessary equipment for capturing CO₂ and introducing it into photobioreactors, mentioning a separation process, and describing a final extraction of biomaterials, although the claims only explain constituent elements of the CO₂ collecting system and their introduction into the photobioreactors, as well as the different photobioreactor options.

Document ES 2370583 A1 discloses photobioreactors and accumulation tanks, without mentioning the rest of the process to obtain biomaterials.

Patent application US 4868123 A relates only to the photobioreactors, but does not introduce the improvements discussed in this patent application that make it much more efficient. This document only relates to a type of photobioreactor for cultivating microalgae which operates differently than that which is disclosed in the present invention, not including a cleaning system or the possibility of all air-water operation, in parallel and crossover. Additionally, it does not mention any sort of subsequent process to that of cultivating microorganisms.

Document ES 2356653 A1 describes an invention with conical photobioreactors that are submerged inside a tank.

Document US 2007048859 A1 includes the steps of cultivating microalgae from the cultivation area with photobioreactors, CO₂ collecting and subsequent treatment of the biomass to obtain a final product based on the biofuel. To this end, it uses photobioreactors without a cleaning system and in a single arrangement, conventional drying systems and a single thermochemical treatment for obtaining biofuel without additional processes for obtaining value-added products. All of this is intended to be carried out without any combination of species or the reuse of rejects.

International patent application WO 2010/021753 A1 discloses a method and a system for obtainig biofuel and biocrude from an algae composition comprising water by hydrotermal process for extracting and/or hydrolizing lipids, to form fatty acids at a predefined temperature and pressure, the water being in a subcritical or supercritical state. The system comprises a separator for partitioning the algae composition into three phases, a first one that is organic, a second one that is aqueous and a third one that is solid. No treating or reciclying step for the rejects produced in the different stages of the method are given in this document.

In light of the aforementioned documents, the present invention aims to improve the efficiency of the processes for obtaining biomass from unicellular algae and extracting value-added products therefrom, by improving on the sequence of the steps of obtainment, separation and extraction, thus making it possible to carry out the process while wet, or to recover the rejects of the process in order to treat them and subsequently reintroduce them into the water and gas lines, as well as by improving the facilities designed for this purpose.

### General description of the invention

The present invention relates to the process for the full conversion of harmful gases, such as greenhouse gases, into products from which subsequent industrial benefit may be derived in the energy and food sectors, both for humans and for animals, by the ultra-intensive cultivation of unicellular algae and with the addition of sea, fresh and/or salty water, sunlight and nutrients, all by means of a closed cycle and making use of all the surplus in each of the stages.

Specifically, the invention is directed at a process for producing biomass and products derived therefrom by cultivating unicellular algae in an aqueous medium supplied with a CO₂ current in an assembly of photobioreactors, comprising:
- injecting a current of sea, salty or fresh water into the photobioreactors as a culture medium for the algae, previously enriched with nutrients, microfiltered and disinfected,
- injecting a strain of at least one species of unicellular algae into the photobioreactors, and placing said strain in contact with the enriched water current under light conditions for the culture to grow by means of photosynthesis;
- injecting a current of gases containing CO₂ into the photobioreactors, characterised in that it further comprises
- extracting part of the algae culture from the photobioreactors, storing in a accumulation tank and sending to a separate unit to extract the biomass by means of mechanical and/or chemical separation, such that the obtained biomass has a humidity degree comprised between 50 % and 90 %;
- subjecting the wet biomass to decantation and/or cavitation in a separate unit in order to extract part of the lipids and/or fatty acids and other added-value products along with a first reject, passing the remaining biomass on to the following step;
- subjecting the remaining biomass from the preceding step to direct, low-temperature, heat liquefaction in a separate unit in order to produce a biocrude and a second reject, all this at a temperature comprised between 250 °C and 350 °C and a pressure comprised between 150 and 210 bar, both limits included, for a time comprised between 1 and 120 minutes;
- recovering the rejects from both the step of lipid, fatty acid and other added-value product extraction and the subsequent step of thermochemical treatment under subcritical conditions, storing each reject in separate chambers and subjecting to physico-chemical treatment based on adjusting salinity, pH, microfiltration, disinfection and addition of nutrients, for subsequent reintroduction into the culture.

Biocrude should be understood as the renewable equivalent of a heavy petroleum-fossil fuel oil, in the present case, however, obtained from algae biomass. Refining biocrude makes it possible to obtain a large variety of compounds, such as naphtha, biofuel and bio-oils, etc.

The present process holds many advantages over the state of the art, mainly the accumulation and subsequent treatment of the algae culture extracted from the photobioreactors in wet conditions (wet biomass), as well as recovering all the rejects or surplus generated in the various mechanical and/or chemical separation processes used to obtain lipids and/or fatty acids and/or added-value products, the subsequent heat treatment and final optimisation process for the end biocrude.

Another improvement resides in the process for treating the biomass once it has been extracted, in order to obtain added-value products such as lipids and fatty acids and other added-value products, such as vitamins, antioxidants such as beta-carotene and astaxanthin, hormones, etc., which may be used in the agro-food, pharmaceutical and cosmetics or biomedical industries, following to guidelines set out in "Selective extraction of carotenoids from the microalga *Dunaliella salina* with retention of viability" (Hejazi MA, de Lamarliere C, Rocha JM, Vermuë M, Tramper J, Wijffels RH; Biotechnol Bioeng. 2002-Jul) or in "Production of cell mass and eicosapentaenoic acid (EPA) in ultra high cell density cultures of Nannochloropsis Sp. (Eustigmatophyceae)" (NingZoy, Chengwu Zhang, Zvi Cohen & Amos Richmond, 2000; European Journal of Phycology, 35:2, 127-133). Lipids, fatty acids and other added-value products are produced before the biocrude is extracted rather than in the same single-step method, as is the case in conventional processes (for example EP 2371940 A1). The method for extracting lipids, fatty acids and other added-value products, using either decantation and/or cavitation means, is carried out prior to the heat liquefaction process, wherein biocrude is extracted. In the present invention, the three processes cited are clearly separated and differentiated from one another and are performed in independent units and machines, which largely optimises the results in comparison with those obtained when the processes are carried out jointly, in addition to enabling the complete recovery of the rejects from the various processes, see "Hydrothermal Treatment (HTT) of Microalgae: Evaluation of the Process as Conversion Method in an Algae Biorefinery Concept" (Laura García Alba, Cristian Torri, Chiara Samori, Jaapjan van der Sped, Daniele Fabbri, Sascha R.A. Kersten and Derk W.F. Brilman (2011). Thermo-Chemical Conversion of Biomass Group, Faculty of Science and Technology, University of Twente, P.O. Box 217, Enschede, The Netherlands).

Furthermore, the fact that this process is carried out in consecutive steps makes it possible to start with a wet extracted biomass, without the need for drying, with a high degree of humidity, in fact, to obtain products such as lipids, fatty acids, added-value products, biocrude and, if possible in preferred cases, finally biofuel.

Recovering waste generated in these processes, for example the first reject produced in the mechanical and/or chemical biomass extraction step, from the steps in which lipids, fatty acids and other added-value elements are extracted, as well as from the reject produced in the steps in which biocrude is obtained, makes a relevant contribution to the development of this kind of methods, since the reintroduction thereof into the process as a raw material, following a prior conditioning process, increases the efficiency of the overall process considerably. The waste obtained in these steps fundamentally, and in by a large percentage, are a culture with a lower concentration of algae after the initial mechanical and/or chemical separation. Their conditioning by physico-chemical treatment thereof is based on the adjustment of salinity, pH, microfiltration, disinfection, addition of nutrients and agitation, for subsequent reintroduction into the culture. Between 50 % and 99 % of culture which is periodically extracted and treated for biomass extraction becomes a reject product for next step and thus is returned to the culture after the prior treatment discussed in the present paragraph.

The present process does not uses antibiotics or fungicides, so that the obtained products have a higher quality that the conventional processes in which they are used (see, for example, international application PTC/ES2007/000733).

Another advantage worth noting is the use of different species in the same biomass production and treatment plant, as a monoculture (each one separated in a different area), such as a lipid-rich species and a second low-lipid species, which complement each other to produce a fuel that is rich in hydrocarbons.

A second object of the present invention consists of a plant for producing biomass and products derived therefrom by cultivating at least one unicellular algae species in aqueous medium supplied with a gas current containing CO₂ in a set of photobioreactors according to the process described in any one of the preceding claims, comprising:
- at least one unit for the collecting, storage and injection of the sea, fresh and/or salty water current connected to a nutrient injection unit that injects nutrients into said current before entering the photobioreactors, and to a microfiltration and disinfection unit;
- at least one unit for the collecting, storage and injection of the gas current containing CO₂ into the photobioreactors;
both units being connected to
- a set of two or more photobioreactors of annular vertical and circular type connected to each other for producing biomass by means of cultivating at least one species of unicellular algae;
characterised in that it further comprises
- means for extracting part of the culture from inside the photobioreactors and an accumulation tank thereof, connected to a mechanical extraction unit and/or to a wet biomass chemical extraction unit;
- a unit for extracting lipids and/or fatty acids and/or other added-value products by means of cavitation, decantation and/or dissolution of the biomass; connected to
- a thermochemical treatment unit for producing biocrude by means of direct, low-temperature, heat liquefaction from the surplus biomass in the unit for extracting lipids and/or fatty acids and/or other added-value products;
- a storage chamber for the first reject produced in the mechanical extraction unit and/or in the chemical extraction chamber, with means for recirculating said reject to the unit for injecting the water current into the photobioreactors after conditioning thereof inside the storage chamber; and
- a storage chamber for the second reject produced in the unit for extracting lipids and/or fatty acids and/or added-value products and in the thermochemical treatment unit, with means for recirculating said reject to the unit for injecting the water current into the photobioreactors after conditioning thereof inside the storage chamber.

The described plant makes it possible to carry out the process object of the present specification optimally, reaching values that are 7 times greater than the surface/volume ratio of the plant, thus increasing the production of biomass by this amount, which amount is higher than those recorded in the state of the art. It has been found that the optimum ratio of the plant described is up to 180 litres/m² of culture in the seeding area as opposed to the 25 litres/m² of culture known in the state of the art, with conventional photobioreactors that are similar to that of the present specification.

### Detailed description of the invention

Regarding the biomass production process, it is worth noting that the gas current may be a current made up entirely of CO₂, or as an alternative may be a mixture of gases from a combustion process.

Optionally, the gas current which is a mixture may be washed, compressed, and the CO₂ may be separated from the rest of the substances contained therein, such as SOx, NOx, and ashes, prior to being introduced into the photobioreactors. This adaptation of the gas current prior to being injected is performed by cooling the gases if needed so that the maximum temperature thereof is always below 40 °C, and separation thereof is performed according to the aforementioned types. Also preferably, but not by way of limitation, gas current may be mixed with compressed air prior to being introduced into the photobioreactors, regardless of whether said current is only CO₂ or a mixture of gases.

In a particular embodiment, the gas current is injected radially into the photobioreactor. Preferably, said injection is performed by means of a diffuser that is integrated between the tubes making up the photobioreactor, in a radial fashion, and covering the entire perimeter of the tube, said diffuser being placed in the mean radius between outer tube and the inner tube with holes drilled in it so as to enable the CO₂ to exit with or without compressed air. Said injection is preferably carried out discontinuously, in pulses with a duration comprised between 1 and 3600 seconds at time intervals comprised between 1 to 3600 seconds, both limits included. This discontinuity, together with the cycles of the cells in the culture, promotes fixing of greenhouse effect gases.

Since both chemical and biological collecting by unicellular algae of the gas current containing CO₂ is between 45 % and 60 %, a system for collecting surpluses that come out the top of the photobioreactors may be incorporated in the plant, for subsequent reintroduction thereof into the plant's gas line via the corresponding plant compressors, mixing tanks and piping and associated control system. The gas collecting efficiency increased up to a value comprised between 60 % and 90 % with this alternative.

Preferably, the gas current is injected by an amount comprised between 0.2 m³/m³ of culture and 2 m³/m³ of algae culture.

After water collection, it is subsequently (micro-) filtered depending on its salt content, especially if the water is seawater, along with removal of organic material and oily products, and micro-filtering of clays and other undesired elements in accordance with the type of unicellular algae being cultivated.

The water current may be enriched with macronutrients, micronutrients and/or trace elements, in a percentage comprised between 10 % and 30 % by weight of the obtained biomass. Specifically, nutrients may be selected from nitrogenised salts such as nitrate and ammonium, phosphates and any combination thereof.

In a preferred embodiment and alternatively to the usual air-lift in the operations of this kind of photobioreactors in terms of the liquid-air flow conditions, the water current can be rotated inside the photobioreactors in the opposite direction to the injection of the bubbles with the gas in question when both are put in contact. This enhances the absorption of the gas by the unicellular algae. It is a chemical effect consisting of the fact that, by opposing the flow of the fluid to that of the CO₂ bubbles, a larger collecting surface is obtained, and absorption yield is improved.

Commonly, but not by way of limitation, the strain is introduced into the plant through one of the photobioreactors, diluting it in the sea, fresh and/or salty water through the existing pump system. It subsequently spreads out from this photobioreactor, 50 % being extracted from the remainder when the concentration is suitable, and seawater is always added to fill out the volume of the photobioreactor.

Preferably, culture is maintained inside the photobioreactors at a temperature comprised between 5 °C and 45 °C, still more preferably between 15 °C and 35 °C.

In the most preferred embodiment, the algal culture is kept inside the photobioreactors until reaching a concentration comprised between 100 million cells/ml and 600 million cells/ml. Thus an amount of daily culture, which is comprised between 2 % and 50 % of the total volume inside the photobioreactors, may be extracted from the photobioreactor. In a more preferred embodiment, said portion of the algal culture is accumulated or stored after extraction with constant stirring and aeration or air and CO₂ supply, which makes it possible to achieve different reactions depending on the characteristics of collected culture.

Mechanical separation of the biomass may be performed by means of one of the processes selected from the group consisting of centrifugation, super-centrifugation, decantation and/or filtration, and chemical separation is performed by means of flocculation. If both mechanical and chemical actions are performed in the process, then each of them is carried out in separate units, such that preferably chemical separation of the biomass is performed first and mechanical separation performed second in a sequential way, thus reducing the level of culture in the mechanical separation process with consequent improvements in process efficiency.

It is worth pointing out that the biomass that is left over from the mechanical and/or chemical separation processes and that of the extraction of lipids and other products, which is used to obtain bio-crude may be comprised between 80 % and 99 % of the total biomass, since in the previous stage only between 1 % and 20 % of total treated biomass has been extracted.

Also in the most preferred embodiment, the first reject, from the biomass separation step, and the second reject, from subsequent processes to obtain lipids and/or fatty acids and/or value-added products and bio-crude are stored under continuous stirring by means of propeller stirrers that ensure complete homogenisation of the product in less than 2 hours, and which may be vertical and/or horizontal, with one or more blades depending on the tank and with aeration or a mixture of air and CO₂, and is conditioned before being reincorporated into the line of water to inject into the photobioreactors.

As a result of reintroducing of rejects of the conditioned form back into the process and as a product generated in certain moments and under certain temperature and aeration conditions by itself, a film of foam is formed on the top layer of the water current inside the photobioreactors. Thus, in an optional embodiment, the foam may be collected, said foam containing biomass and/or nutrients in a percentage comprised between 1 % and 5 % of the total existing volume in the photobioreactors.

In a preferred embodiment of the process, the strain of unicellular algae is selected from the group consisting of eukaryotic microalgae and in particular Cyanobacteria, Chlorophyceae, Rhodophyceae, Eustigmatophyceae, diatoms, Dinophyceae, Bacillariophyceae, Xanthophytes and Phaeophyceae, and any combination thereof.

In the most preferred embodiment, bio-crude is subjected to cavitation, decantation, pressing and/or heat treatment in a separate unit to obtain biofuel (distilled and purified bio-crude). The reject of this step can also be stored, treated and reintroduced into the process like the other rejects and in the same conditions described for them.

With regard to the biomass production plant described in the previous section, it is worth noting that the gas capturing unit can be nourished by a current of greenhouse gases, for example coming directly from the smoke stacks of the industrial facilities that emit them. This unit is preferably formed by take-offs from smoke stacks, duct networks, automatic operating systems, accessories and extractors.

The plant may further comprise a compressed air injection unit connected to the unit for collecting, storage and cleaning, and injection of the current of gases containing CO₂, prior to entering the photobioreactors. The plant may further comprise an accumulation unit for the gases to be injected, so that if the main supply source thereof fails, the process may continue. The capacity of these tanks is such that they allow normal operation of the plant without the usual supplies from one month to four months.

The water current collecting, storage and injection unit is made of the consequent systems of pipes, electric pumps and filters, as well as control elements. Preferably, the accumulation tank of sea, fresh and/or salty water has a capacity of between two sixths and four sixths of the total capacity of the culture volume of the plant. The control elements are pH and temperature probes, a conductivity probe, turbidimeters and maximum and minimum levels. This, together with the electrically operated valves associated to the plant management system, enables the sea, fresh and/or salty water that is to be introduced into the feeder tanks of the photobioreactors enter in the ideal conditions.

Optionally, the plant comprises from 2 to 5000 photobioreactors. Said photobioreactors are preferably constituted by an outer tube and an inner tube delimiting a space between them, wherein the water and gas currents are injected and where the unicellular algae are cultivated, said space being divided into longitudinal odd number sections from 1 to 9, and the span of the passage inside the tubes being smaller than 5 cm The tubes may have a height equal to or greater than 10 meters. Because the tubes are divided into 3, 5, 7 or 9 longitudinal sections or compartments, the functioning of the light-dark binary cycles is exactly the same throughout the whole section of the tube, therefore achieving much greater efficiency and more control than that which is achieved with the different areas present in a normal cylindrical tube. The inner and outer tubes may preferably be made of plastic materials, both rigid and flexible, with the particular feature that the inner tube may or may not be translucent and that the inner cavity may or not may be used to adjust the temperature of the existing culture in the ring.

Even more preferably, the photobioreactors further comprise a translucent concentric tube covering the outer wall of the outer tube. This tube made of transparent material prevents the sun from shining directly onto the outer tube containing the culture and thereby may prevent the photoinhibition effects that occur with high levels of irradiation and greatly reduce the energy requirements for adjusting the temperature of the culture, as well as number of other associated improvements such as reduction in thickness, in turn lowering the production cost of the tubes.

In a particular embodiment of the invention, the set of the photobioreactors comprises:
- an area for mixing the gas current, the water current and a strain of the algae to be cultivated;
- an area for growing the algae culture; and
- an area for extracting the culture from inside the photobioreactors.

Each area is comprised of specialised photobioreactors for each function, the three areas are connected together in the logical order: the mixing area is connected to the culture growing area and, in turn to the extraction area. That is, the plant has photobioreactors which are used for the mixing or feeding, others which are used for growing, and other final ones for the culture collection. This is achieved with the different arrangements of the tubes inside each photobioreactor, thus varying the separation between them, water-sheet thickness, culture conditions and a number of other parameters allowing for optimal sequential development to optimise the process, as has been explained above.

The photobioreactors may be connected to each other by means of pipes buried under ground where the plant is located, the water current with the culture being driven from one photobioreactor to another by the electric pump.

The photobioreactors may further comprise electric pumps which are associated to all the network of pipes that interconnect the cultivation area, containing a pump for each group of photobioreactors. With the electrically operated valves installed in the pump zone, it is possible to use the controls to run the system explained in the preceding paragraph and/or in the present one and all others that may be possible. Each one of these pumps makes the water spin in the opposite direction to that of the gas current injection when both are put in contact. In another alternative operating option, the same pump is the one which recirculates the culture through the buried pipes, thus favouring the heat exchange with the ground (at a constant temperature) and thus achieving the highest productivity of the process with the lowest possible energy consumption. In this case, culture recirculation varies between 10 % and 60 % of the unitary volume.

The arrangement of the tubes in the set of the photobioreactors shall be that which enables maximal development of the unicellular algae depending on their degree of concentration with respect to the irradiation and temperature at that time. Since these parameters oscillate both through the year and depending on the location of the plant, different alternative designs are combined, while other parameters also vary, such as residence time in each of the configurations, daily extraction percentage, the percentage of recirculation flow over the total volume and nutrient and CO₂ supply, in order to obtain the best values for reproduction. Specifically, the arrangement of the tubes or columns in the photobioreactors and in the cultivation area is adapted to the different needs of the culture depending on the time of year and environmental conditions. There are, in the state of the art, photobioreactors formed by tubes with a linear arrangement, others with circular arrangements, and others with double arrangements of these both, such that the distances between the components also vary, thus combining the different possibilities of irradiation/passage span/speed of CO₂ and air mixture, to always obtain the best production. In the case of the present invention, as a consequence of the division into areas of the algal culture, the plant is stratified according to the environmental conditions and the different reproductive phases of the culture, so that the plant may be adapted to each time of the year, achieving the best possible efficiency. Irradiation needs in one time of the year are very different from those required in other time, for which reason the path of the culture is adapted from its initial seeding to extraction, and between one extraction and the next. That which has been indicated corresponds to the principles demonstrated in the technical article "Design principles of photo-bioreactors for cultivation of microalgae", by D. Clemens Posten, Institute of Life Science Engineering, Division of Bioprocess Engineering, University of Karlsruhe, Strasse am Forum 8, D-76131 Karlsruhe (Germany), DOI: 10.1 002/elsc.200900003, 29 May 2009, and in "Microalgal Photobioreactors: Scale-up and optimization", Barbosa, M.J.G.V., 2003.

Optionally and preferably, the photobioreactors further comprise an automated device for cleaning the walls of the tubes delimiting the space between the inner tube and outer tube, said cleaning device being constituted by an elastomeric scraper whose plan corresponds to the profile of the space between the tubes and a thickness comprised between 2 and 5 cm, with a central through hole that has a lower frustoconical section and an upper cylindrical section, the hole having the overall shape of an inverted funnel; and a sphere made of plastic material with a larger diameter than the diameter of the cylindrical section of the hole, which moves inside the space defined by the lower frustoconical section of the through hole. The incorporation of this cleaning system allows the plant to operate without the need to empty the photobioreactors, which simplifies maintenance of the facility and efficiency thereof with respect to the prior art. The cleaning device operates as follows:
1.- In the normal position it is at rest at the bottom of the tube, in the cultivation area, i.e., between the outer and the inner tubes.
2.- Once it becomes desirable to activate it, the flow direction of the culture is changed from its normal operation (the culture enters through the top part and is collected at the bottom) to the opposite direction, and should the operation being used that in which it is fed from below and spills over the top, the flow rate is increased so that the device is activated once it is enough to make the inner sphere rise.
3.- The operating time of the device, which is activated because of the changes to the electrically operated valves found in the area of each of the culture pumps, is such that it makes it possible to make the cleaner go upwards without butting up against in the top cover of the photobioreactor.
4.- Once the cleaner reaches the top, this option is inhibited, such that it returns to the previous state, and falls under its own weight (its density is greater than that of the culture water) back to its original position.

The operation may be repeated from 1 to 50 times a day depending on the internal cleaning state of the tubes, which in turn depends on the species being employed and the conditions under which it is being cultivated.

Ultimately, annular vertical tubes are used as photobioreactors, it being possible for a third outer layer to be arranged in different ways inside the photobioreactors, which enable the cultivation area to adapt to existing climatic conditions in each period, differentiating culture recovery areas, mixing areas and the various growth areas prior to the extraction area. This equally results in maximum CO₂ absorption being attained in each one of the current environmental conditions. These aspects, in addition to improving both the collection and reproduction efficiencies of the unicellular algae, improve the overall performance of the system, preventing *fooling* (prokaryotes and unicellular algae stuck to the outer tube) from forming and largely reducing maintenance operations owing to the incorporation of a cleaning system for the tubes, without it being necessary to empty the same.

As described above, a foam film may be form on the upper layer of the water current inside the photobioreactors, especially as result of the reintroduction of the reject from the various biomass treatment stages, until the end products desired are obtained. This foam film may even be generated naturally inside the photobioreactors, in certain conditions and at certain times during the reproduction process, even prior to introducing the treated rejects, which contain a high percentage of nutrients and/or biomass. For this reason, the photobioreactors may further comprise means for collecting the foam, based on an upper channelling of the appropriate dimensions according to the volume of the photobioreactor, conveniently driven to an accumulation area generated for this purpose in the plant and drive means (pipes and electric pumps), which drive said foam to the foam accumulation tank for foam coming from the culture extracted from the photobioreactor. It will subsequently be reintroduced back into the photobioreactors supply tank or to the clarification tank, according to the treatment applied and depending on the unicellular alga being cultivated.

The top portion of the photobioreactors may also comprise means for collecting the excess CO₂ not consumed by the algae culture and means for driving said excess gas from the gas collecting and injecting unit to the photobioreactors, in order for them to be reintroduced.

The capacity of the unit for accumulating the culture extracted from the photobioreactors is preferably comprised between half and one tenth of that of the total volume of the plant. It may also have stirrers and supply means with CO₂ and/or compressed air and measurement and control systems, to be integrated into the plant operation. The control elements include pH and temperature probes, conductivity probe, turbidimeters, maximum and minimum levels and nutrient control probes.

In order to attain optimal biomass production, the storage units for the first and second reject may comprise stirring and aeration means or means for injecting air and CO₂ inside, in order to improve the behaviour of the culture and/or the subsequent reincorporation into the cultivation area thereof, it being possible to produce decantation and/or variations in the composition as required. The capacity of each of the reject storage units is normally between half and one tenth the capacity of the total volume of the plant. Optionally and preferably, when both rejects have been treated in their respective storage units, they may be driven to a joint accumulation chamber, where all the rejects treated come together prior to being reintroduced into the process. This accumulation unit for treated rejects is connected at its input to the output in the rejects storage and treatment units, and at its output to the water lines of the plant (means for injecting sea, fresh and/or salty water into the photobioreactors).

The photobioreactors may also comprise means for controlling the temperature, which are preferably joint temperature and pH probes very common in the state of the art. They are integrated in line in the network of pipes that carries each photobioreactor, in order to join the different tubes and between one photobioreactors and others, by means of heat pumps of the type condensed by air, by water and/or are geothermal, in order to keep said parameter between 5 °C and 45 °C, as required by the process. Said temperature control or adjustment means are made up of heat pumps of the type condensed by air, condensed by water and/or are geothermal, in order to keep culture in the desired temperature conditions. With the addition of the third concentric outer tube, alongside the two inner tubes between which the culture is found, temperature adjustment needs are reduced considerably, this being one of the aspects that improve the overall performance of the system.

Additionally, the plant may incorporate cleaning and disinfection means, which are arranged in line in the network of pipes and electric pumps responsible for the repositioning and extraction processes for the culture of the photobioreactors, as well as in the rejects recovery processes and exchanges in the different tanks, being selected from the group consisting of ultraviolet rays, active carbon filters and/or ozonation, thus meaning no contaminant element is poured to the environment. also as differentiation, as well as means for collecting accidental leaches and spills, formed by driving means and pumping means up to the storage unit for the reject extracted in all the processes for obtaining biomass, from the units for extracting lipids and/or fatty acids and/or added-value products and the thermochemical treatment units. The cleaning and disinfection means are incorporated into the entire pipe system and remaining system elements. These systems differ from the usual ones used up until now, since they do not emit any toxic substance into the environment. The plant, in order to collect accidental leaches and spills, comprises networks of underground pipes, catch basins and electric pumps, which in the event of the culture accidentally being spilt, will be sent to the corresponding reject deposits in order to be treated and subsequently reused in the culture.

The plant optionally but not exclusively comprises means for controlling the process formed by sensors, flow meters, electrically operated valves and common elements in an SCADA (Supervisory Control and Data Acquisition) system. These elements are located in the networks of pipes and tanks and are responsible for the independent operation of the plant, according to the operating conditions thereof and owing to the electrically operated valves and probes installed.

Finally, in the most preferred of all embodiments, the plant described in any of the variations thereof also includes a biofuel production unit and a rejection by means of cavitation, decantation, pressing and/or heat treatment for the biocrude obtained in the thermochemical treatment unit in which said bio-crude is obtained, to which it is connected. This unit may also be connected to a reject storage unit, which works and is connected to the system thereof, in the same conditions as the storage and conditioning unit for the first and second rejects.

Ultimately, the installation described, wherein a wide range of products of commercial interest are generated from biomass, operates as a biorefinery.

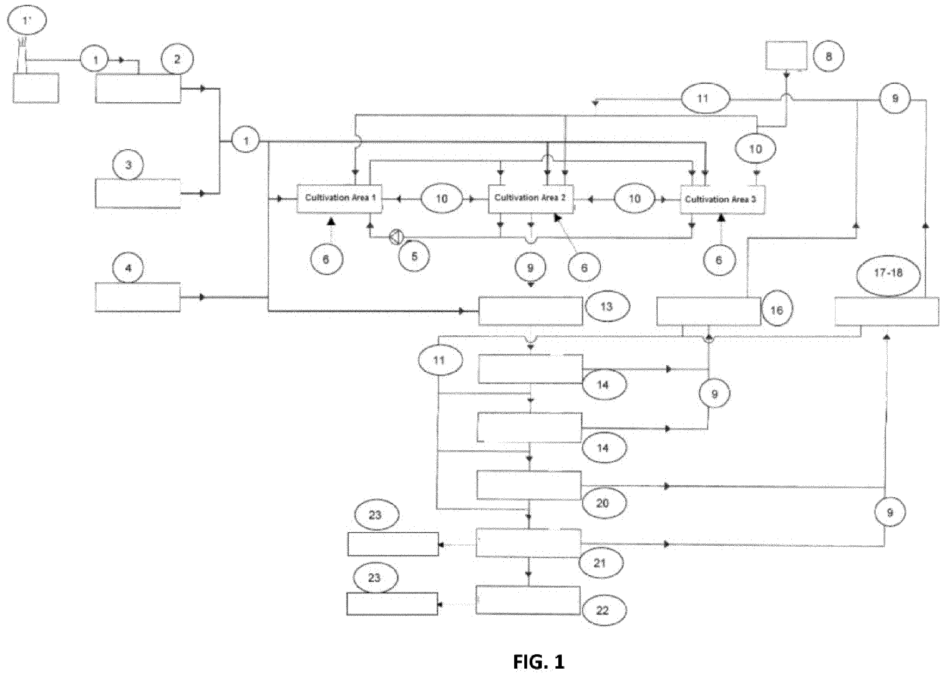

As described above and as shown in Figure 1, the biomass production plant comprises:
(1) a unit for collecting, storage and injection of greenhouse gasses coming from smoke stacks of industrial facilities that emit them (1'). This system is formed in a non-limiting manner by take-offs from smoke stacks, duct networks, automatic operating systems, accessories and extractors.
(2) a unit for washing and compressing greenhouse gases, the temperature of which is adjusted if the same is above 40 °C.
(3) a unit for mixing and injecting the greenhouse gases with air, as well as with gases returned from the thermochemical processes and/or surplus of those introduced into the photobioreactors that are consumed in the plant.
(4) A unit for accumulating greenhouse gases, in such a way that if the main supply source thereof fails, the process may carry on. The capacity of these tanks is such that they enable the plant to operate normally, when usual supplies are not provided from between one to four months.
(5) An electric pump in each group of between 2 and 5000 photobioreactors, which makes the fluid in the photobioreactor spin in the opposite direction to that in which the bubbles, with the indicated gasses injected, are introduced;
(6) A set of between 2 and 5000 annular vertical photobioreactors made from plastic materials. In all cases, the photobioreactor has an inner span of less than 5 cm and the ratio achieved by the same in terms of its design and volume (m³)/ surface area (m²) ratio. This ratio may reach up to 180 litres/m². Each photobioreactor is made up of two tubes, one inner tube and one outer tube, between which the algae are cultivated. Outside the two tubes between which the culture is found, a translucent tube is placed, which prevents the direct irradiation of the outer tube containing the culture. Moreover, the section between the inner tube and outer tube containing the culture is divided into X number of longitudinal sections
(7) An automatic tubes cleaning device containing the culture, as described in the previous section.
(8) A unit for collecting, storage and injection and subsequent filtration of sea, fresh or salty water by means of piping systems, electric pumps and filters, as well as control elements. It also has an additional supply water accumulation tank, with a capacity of between one sixth and two sixths of the total capacity of the plant culture volume.
(9) A system of pipes that interconnect the cultivation areas to the culture accumulation and treatment areas and finished product accumulation and treatment areas.
(10) A unit for injecting nutrients into the water current that supplies the photobioreactors, in order to promote the rapid development of unicellular algae. There is a dosage cap for macro and micronutrients and trace metals. Owing to the nutrient probes installed in line in the piping of the process, the corresponding orders will be given to the nutrients pumps, so that they inject the culture as required.
(11) Means for cleaning and disinfecting the plant by means of ultraviolet rays and active carbon filters, in addition to ozonation. They are introduced into all the piping systems and remaining elements in the plant.
(12) Means for collecting upper foam in each one of the photobioreactors. This foam is collected and sent by means of the corresponding system of pipes and electric pumps to the corresponding accumulation tanks, thereby being reintroduced into the process.
(13) Unit for accumulating the culture extracted from the photobioreactors. The capacity thereof is between half and one tenth that of the total volume of the plant. It has stirrers and a supply system with CO₂ and/or compressed air and measurement and control systems, to be integrated into the plant operation, thus making it possible to attain different reactions therein, according to the characteristics of the culture collected.
(14) Mechanical and chemical separation units for separating the unicellular algae culture extracted and accumulated in the unit (13).
(15) Means for controlling the temperature, made up of heat pumps of the type that are condensed by air, in order to keep the culture in temperature conditions of between 5 °C and 45 °C, as required.
(16) Chamber for accumulating rejects from the mechanical and chemical separation process. Between 50 % and 99 % of the culture volume filtered is returned to these deposits for treatment and subsequent reincorporation into the cultivation process. Its capacity is between half and one tenth that of total volume of the plant. They have stirrers and aeration systems and/or CO₂+air input, and measurement and control systems to be integrated into the plant operation.
(17) Chamber for accumulating the rejects generated in the units for extracting lipids and/or fatty acids and/or value-added products and for obtaining bio-crude. All those elements considered to result from the various processes, which do not have an end use, are sent to this tank and integrated back into the system after having undergone corresponding treatment, pH and salinity adjustment, microfiltration, disinfection, nutrient addition and agitation. Their capacity is between half and one tenth that of the plant's total volume. They have stirrers and aeration systems and/or CO₂+air input, and measurement and control systems to be integrated into the plant operation.
(18) Means for collecting accidental spills and leaches in the plant. In the event of this embodiment, the unit in which the spills and leaches are accumulated is the same as the reject accumulation unit. It has a system of underground pipe networks, catch basins and electric pumps. In the event of the culture accidentally being spilt, it is sent to the corresponding unit. This is a tank for rejects to be treated and subsequently reused in the culture. In the event of the present embodiment, said means for collecting and storing spills are the same as those for accumulating rejects generated in the units for extracting lipids and/or fatty acids and/or added-value products.
(19) Means of driving the water currents, culture and products obtained in the process. Said means are electric pumps, which perform all movements of the culture and subsequent products between the different zones described in the above elements.
(20) Unit for extracting lipids and/or fatty acids and/or added-value products.
(21) Thermochemical treatment unit for producing biocrude.
(22) Unit for producing biofuel by means of treating biocrude.
(23) Biocrude and biofuel accumulation tanks.
(24) General plant control means, encompassing all the sensors, flow meters, electrically operated valves and other conventional elements in a SCADA type system, which make it possible to vary the operating conditions of the plant within the different values established for each one of the parameters.

### Description of the drawings

**Figure 1****.** Illustrative diagram of the biomass production plant in accordance with the present invention, according to the preferred embodiment described in the previous section.

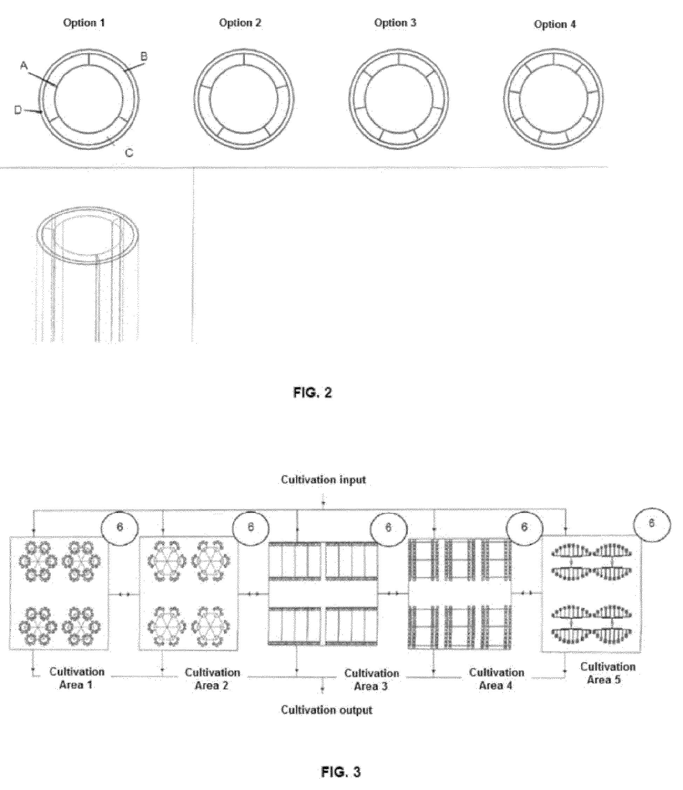

**Figure 2****.** Plan view (and a view in 3D) of alternative configurations of tube photobioreactors to be used in the production plant depending on the number of longitudinal sections into which they are divided: Option 1 with 3 sections, Option 2 with 5 sections; Option 3 with 7 sections and Option 4 with 9 sections. A= Inner tube; B= Outer tube; C= Culture; D= Ventilation tube.
**Figure 3****.** Alternative set of photobioreactors. Different arrangements of the tubes containing the culture, at the time a photobioreactor is formed, are shown in this figure, in such a way that the distances between the same vary, and, depending on their orientation and the placement of a photobioreactor relative to contiguous photobioreactors, first several irradiation values are reached, thereby obtaining an optimised plant suited to all conditions throughout the year depending on its location.

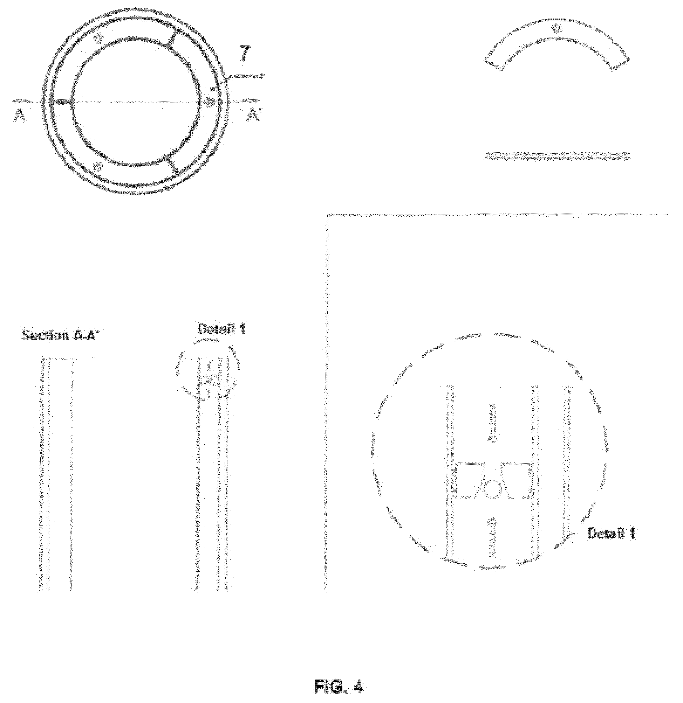

**Figure 4****.** Plan, elevation and transverse views of the cleaning device incorporated inside the photobioreactors tubes.

### Examples

Three examples of the present invention are described below, which in no case can be considered limiting examples of the invention.

### Example 1:

In Figure 1, the plant designed and the entire process object of the present invention are described. Starting with an estimated cultivation volume of 100 m³, the process is initiated by introducing these 100 m³ of seawater through the mandatory seawater collection system (8). After having disinfected and filtered (11) the same, it is introduced into the cultivation photobioreactor (6), all of the above being carried out at a grade of filtration of less than 1 micron.

The initial strains for inoculating the biomass are also filtered and disinfected (11) prior to being introduced into the photobioreactors (6).

The cultivation area operating system is carried out progressively, in such a way that the photobioreactors (6) are firstly filled with less water thickness sheet, and therefore more productivity, and it is subsequently spread out as the optimal concentration is achieved (between 100 M of cells/ml and 600 M of cells/ml) until the entire cultivation area is obtained, at which point the rest of the process may be started. This step may take from several days to several weeks depending on environmental conditions and the volume and concentration of the initial strain used to begin with.

During this process, and continuously during normal exploitation of the plant, CO₂ is introduced from the gas collection system (1), through its injection means, to the photobioreactors (6), after having been washed in the corresponding unit (2). The amount of gas and compressed air mixture to be introduced depends on the environmental conditions and the state of the culture, however it will be found between the values of 0.2 m³ of air+CO₂ per cubic meter of culture and 2 m³ of air+CO₂ per cubic meter of culture. As mentioned above, the introduction of air into the system does not necessarily have to be continuous. In an alternative scenario, the culture recirculation pump (5) will act in parallel to the introduction of CO₂ and/or CO₂+air or alone, if said supply were not necessary, given the state of the culture at certain points in time. The overall ratio of CO₂ removed is 1.8 kg/CO₂ per each kg of biomass subsequently produced. Auxiliary CO₂ tanks (4) are anticipated in the state of the art, which may make up for any lack of CO₂ through the usual collection system (1).

Meanwhile, nutrients (10) are introduced into the culture, which are mainly nitrogenous salts (nitrate and ammonium) and phosphates, in the proportion required according to the needs and level of development of the same. The total proportion of nutrients added is between 10 % and 30 % of the biomass obtained. The photobioreactors system has been equipped with an upper foam collection system, in such a way that the nutrients and biomass present in this area are exploited (12).

A culture temperature adjustment system (15) has been installed, although, with the abovementioned improvements made to the photobioreactors, in terms of incorporating a third outer tube, the use thereof will not be necessary during 98 % of the plant operation period. The working temperature ranges greatly, preferably being at between 15 °C and 35 °C. Moreover, an accidental spill collection system (18) has been installed, which spill would be introduced back into the system after appropriate treatment (11) thereof.

Once the optimal degree of culture concentration has been achieved, a daily extraction comprised between 2 % and 50 %, preferably 25 % of the total culture, begins, i.e., between 2 and 50 m³ (9). Meanwhile, despite the fact that most of the water that returns to the cultivation areas, 2 % of that which is extracted, is supplied as new water through the seawater collecting system (8).

The culture, at optimal concentrations, is taken to the accumulation tanks (13). This step feeds the decantation/filtration and/or centrifugation equipment (14) which return part of the culture, at between 50 % and 99 % of the total, to the rejection tanks (16), in order for them to be subsequently treated and introduced back into the culture (6). After this stage, a biomass is obtained, which has an approximate weight of between 40 kg/day and 110 kg/day and a humidity of between 50 % and 90 %, suitable for the subsequent introduction thereof into the next process, without any drying process being required.

The next step of the process is extracting lipids and/or fatty acids and/or added-value products (20), in which between 4 kg/day and 10 kg/day of added-value products are produced.

This is followed by the thermochemical transformation process (21), in which between 20 % and 60 % of bio-crude, between 10 % and 30 % of water, between 1 % and 10 % of char and between 1 % and 30 % of various gases are obtained. This step can be performed with or without a catalyst, and the conditions range between 250 °C and 350 °C and between 150 bar and 210 bar. The processing time can vary between 1 minute and 120 minutes. After this process, a portion of the resulting products are sent back to tanks to be treated and subsequently introduced into the process, and the portion of product obtained as bio-crude goes on to the final conversion to biofuel treatment (22). Different tanks (23) accumulate the products obtained in one or another process prior to their final destination. Finally, between 20 and 50 kg/day of bio-crude are obtained, with a CO₂ conversion of between 72 and 198 kg/day.

### Bibliographic references

- EP 2371940 A1: "Producing Bio-oil by providing a gas having high carbon dioxide content, splitting the current of the gas, adding nitrogen-rich and/or phosphorus-rich nutrients to the sea and/or fresh water, and contacting with an algal culture". Cia Ciacomo& Alessandro Concas.
- "Design principles of photo-bioreactors for cultivation of microalgae", D. Clemens Posten, Institute of Life Science Engineering, Division of Bioprocess Engineering, University of Karlsruhe, Strasse am Forum 8, D-76131 Karlsruhe (Germany), DOI: 10. 1 002/elsc.200900003, 29 May 2009.
- "Microalgal Photobioreactors: Scale-up and optimisation", Barbosa, M.J.G.V., 2003.
- "Selective extraction of carotenoids from the microalga Dunaliella salina with retention of viability" (Hejazi MA, de Lamarliere C, Rocha JM, Vermuë M, Tramper J, Wijffels RH; Biotechnol Bioeng. 2002-Jul).
- "Production of cell mass and eicosapentaenoic acid (EPA) in ultra high cell density cultures of Nannochloropsis sp. (Eustigmatophyceae), NingZoy, Chengwu Zhang, Zvi Cohen & Amos Richmond (2000), European Journal of Phycology, 35:2, 127-133.
- "Hydrothermal Treatment (HTT) of Microalgae: Evaluation of the Process as Conversion Method in an Algae Biorefinery Concept" (2011). Laura Garcia Alba, Cristian Torri, Chiara Samori, Jaapjan van der Sped, Daniele Fabbri, Sascha R.A. Kersten, and Derk W.F. Brilman. Thermo-Chemical Conversion of Biomass Group, Faculty of Science and Technology, University of Twente, P.O. Box 217, Enschede, The Netherlands.
- US 2008086938 A1: "Photosynthetic Carbon Dioxide Sequestration and Pollution Abatement". Suresh M Menon, David E Newman, Jagadish Chandra Sircar, Kashinathan Alisala, Kay A Yang, Samantha Orchard, Sara Guidi.
- WO 2007147028 A2: "Method and Apparatus for CO₂ sequestration". Malcom Glen Kertz.
- ES 2370583 A1: "Fotobiorreactor para el cultivo en continuo de algas unicelulares y sistema modular que comprende dichos fotobiorreactores" Spanish High Council for Scientific Research (CSIC).
- US 4868123 A: "Apparatus for the intensive, controlled production of microorganisms by photosynthesis". Xavier Berson, Michel Bouyssou, Yves Castel, Daniel Chaumont, Claude Gudin.
- ES 2356653 A1: "Fotobiorreactor para el cultivo de organismos fotótrofos" Universidad de Cantabria.
- US 2007048859 A1: "Closed system bioreactor apparatus". James T. Sears.
- PCT/ES2007/000733: "Fotobiorreactor Electromagnético para la obtención de biomasa" BiofuelSystems, s.l.
- WO 2010/021753 A1: "Systems and methods for hidrotermal conversion of algae into biofuel". LIVE-FUEL INC.

## Claims

1. A process for producing biomass and products derived thereof by means of cultivating unicellular algae in aqueous medium fed with a CO₂ current in a set of photobioreactors, comprising:
- injecting a current of sea, salty or fresh water into the photobioreactors as a culture medium for the algae, previously enriched with nutrients, microfiltered and disinfected,
- injecting a strain of at least one species of unicellular algae into the photobioreactors, and putting said strain in contact with the enriched water current under light conditions for the culture to grow by means of photosynthesis;
- injecting a current of gases containing CO₂ into the photobioreactors,
**characterised in that** it further comprises
- extracting part of the algae culture from the photobioreactors, storing in a accumulation tank and sending to a separate unit to extract the biomass by means of mechanical and/or chemical separation, such that the obtained biomass has a humidity degree comprised between 50 % and 90 %;
- subjecting the wet biomass to decantation and/or cavitation in a separate unit in order to extract part of the lipids and/or fatty acids and other added-value products along with a first reject, passing the remaining biomass on to the following step;
- subjecting the remaining biomass from the preceding step to direct, low-temperature, thermal liquefaction in a separate unit in order to produce a biocrude and a second reject, all this at a temperature comprised between 250 °C and 350 °C and a pressure comprised between 150 and 210 bar, both limits included, for a time comprised between 1 and 120 minutes;
- recovering the rejects from both the step of lipid, fatty acid and other added-value product extraction and the subsequent step of thermochemical treatment under subcritical conditions, storing each reject in separate chambers and subjecting to physicochemical treatment based on adjusting salinity, pH, microfiltration, disinfection and addition of nutrients, for subsequent reintroduction into the culture.

2. The process according to the previous claim, wherein each one of the rejects is stored in a separate unit under continuous stirring and aeration or with a mixture of air and CO₂ before reincorporation.

3. The process according to any one of the previous claims, wherein the gas current is a CO₂ current or a mixture of gases from a combustion process.

4. The process according to any one of the previous claims, wherein the gas current is cooled, washed, compressed and the CO₂ is separated from the other gases prior to introduction thereof into the photobioreactors.

5. The process according to any one of the previous claims, wherein the gas current is mixed with compressed air prior to introduction thereof into the photobioreactors.

6. The process according to any one of the previous claims, wherein the gas current is injected in a radial and discontinuous manner into the photobioreactor, in pulses lasting between 1 and 3600 seconds at time intervals comprised between 1 and 3600 seconds, both limits included.

7. The process according to any one of the previous claims, wherein the gas current is injected in an amount comprised between 0.2 m³/m³ of culture and 2 m³ /m³ of culture.

8. The process according to any one of the previous claims, wherein the nutrients with which the water current is enriched are macronutrients, micronutrients and/or trace elements, in a percentage comprised between 10 % and 30 % by weight of obtained biomass.

9. The process according to the previous claim, wherein the nutrients are selected from nitrogenous salts such as nitrate and ammonium, phosphates and any combination thereof.

10. The process according to any one of the previous claims, wherein the water current is made to spin inside the photobioreactors in the opposite direction of the injection of gas current when the two of them are put into contact.

11. The process according to any one of the previous claims, wherein the culture is kept inside the photobioreactors at a temperature comprised between 5 °C and 45 °C.

12. The process according to any one of the previous claims, wherein the algae culture is kept inside the photobioreactors until a concentration comprised between 100 million cells/ml and 600 million cells/ml is reached.

13. The process according to any one of the previous claims, wherein an amount of culture comprised between 2 % and 50 % of the total volume inside the photobioreactors is extracted daily from the photobioreactors.

14. The process according to any one of the previous claims, wherein the mechanical separation for obtaining the biomass is carried out by means of one of the processes selected from the group consisting of centrifugation, super-centrifugation, decantation and/or filtration, and the chemical separation is carried out by means of flocculation.

15. The process according to any one of the previous claims, wherein a foam that is formed on the upper layer of the water current inside the photobioreactor as a consequence of the reintroduction therein of the treated reject, is collected, said foam containing biomass and/or nutrients in a percentage comprised between 1 % and 5 % of the total volume existing in the photobioreactors.

16. The process according to any one of the previous claims, wherein the algae strain is selected from the group consisting of Cyanobacteria, Chlorophyceae, Rhodophyceae, Eustigmatophyceae, diatoms, Dinophyceae, Bacillariophyceae, Xanthophytes and Phaeophyceae, and any combination thereof.

17. The process according to any one of the previous claims, the biocrude is subjected to cavitation, decantation, pressing and/or thermal treatment in a separate unit in order to obtain biofuel and a reject.

18. The process according to the previous claim, wherein the reject that is obtained along with the biofuel is led to an storage unit under continuous stirring and with aeration or with a mixture of air and CO₂, and is subjected to physicochemical treatment based on adjusting salinity, pH, microfiltration, disinfection and addition of nutrients, for subsequent reintroduction into the culture.

19. A plant for producing biomass and products derived therefrom by cultivating at least one unicellular algae species in aqueous medium supplied with a gas current containing CO₂ in a set of photobioreactors according to the process described in any one of the previous claims, comprising:
- at least one unit for collecting, storage and injection of the sea, fresh and/or salty water current connected to a nutrient injection unit that injects nutrients into said current before entering the photobioreactors, and to a microfiltration and disinfection unit;
- at least one unit for collecting, storage and injection of the gas current containing CO₂ into the photobioreactors;
both units being connected to
- a set of two or more photobioreactors of the annular vertical and circular type connected to each other for producing biomass by means of cultivating at least one species of unicellular algae;
**characterised in that** it further comprises
- means for extracting part of the culture from inside the photobioreactors and an accumulation tank thereof, connected to a mechanical extraction unit and/or to a wet biomass chemical extraction unit;
- a unit for extracting lipids and/or fatty acids and/or other added-value products by means of cavitation, decantation and/or dissolution of the biomass, connected to
- a thermochemical treatment unit for producing biocrude by means of direct, low-temperature, thermal liquefaction from the surplus biomass in the unit for extracting lipids and/or fatty acids and/or other added-value products,
- a storage chamber for the first reject produced in the mechanical extraction unit and/or in the chemical extraction chamber, with means for recirculating said reject to the unit for injecting the water current into the photobioreactors after conditioning thereof inside the storage chamber; and
- a storage chamber for the second reject produced in the unit for extracting lipids and/or fatty acids and/or added-value products and in the thermochemical treatment unit, with means for recirculating said reject to the unit for injecting the water current into the photobioreactors after conditioning thereof inside the storage chamber.

20. The plant according to the previous claim, wherein the storage units of the permeate and reject comprise stirring means and means for aeration or for injecting air and CO₂ inside of it.

21. The plant according to any one of the claims 19 or 20, which further comprises a compressed air injection unit connected to the collecting, storage and injection unit of the gas current containing CO₂ prior to entering the photobioreactors.

22. The plant according to any one of the claims 19 to 21, wherein each photobioreactor further comprises and electric pump that makes the water spin in the opposite direction to that of the gas current injection when both are put in contact inside the photobioreactors.

23. The plant according to any one of the claims 19 to 22, comprising between 2 and 5,000 photobioreactors.

24. The plant according to any of the claims 19 to 22, wherein the photobioreactors are constituted by an outer tube and an inner tube delimiting a space between them in which the water and gas currents are injected and where the algae are cultivated, said space being divided into longitudinal odd number sections from 1 to 9, and the span of the passage inside the tubes being smaller than 5 cm.

25. The plant according to any of the claims 19 to 22, wherein the set of photobioreactors is configured into three areas defined as follows:
- an area for mixing the gas current, the water current and a strain of the algae to be cultivated, which is constituted by one group of photobioreactors; connected to
- an area for growing the algae culture, which is constituted by a second group of photobioreactors; and
- an area for extracting the culture from inside the photobioreactors, which is constituted by a third group of photobioreactors.

26. The plant according to any one of the claims 24 or 25, wherein the photobioreactors further comprise an automated device for cleaning the walls of the tubes delimiting the space between the inner tube and outer tube, said cleaning device being constituted by an elastomeric scraper whose plan corresponds to the profile of the space between the tubes and a thickness comprised between 2 and 5 cm, with a central through hole that has a lower frustoconical section and an upper cylindrical section, the hole having the overall shape of an inverted funnel; and a sphere made of plastic material with a larger diameter than the diameter of the cylindrical section of the hole, which moves inside the space defined by the lower frustoconical section of the through hole.

27. The plant according to any of the claims 19 to 26, wherein the photobioreactors further comprise a translucent concentric tube covering the outer wall of the outer tube.

28. The plant according to any of the claims 19 to 27, wherein the photobioreactors further comprise means for collecting a foam that is formed on the upper layer of the water current, consisting of piping at the top of the photobioreactor, and means for leading said foam to the accumulation tank for the culture extracted from the photobioreactor, which are pipes and electric pumps.

29. The plant according to any of the claims 19 to 28, wherein the photobioreactors comprise in their upper part means for collecting of surplus CO₂ not consumed by the algae culture and means for leading said surplus gas to the gas unit for collecting and injecting gasses into the photobioreactors.

30. The plant according to any of the claims 19 to 29, wherein the photobioreactors are connected to each other by means of pipes buried under ground where the plant is located, the water current with the culture being driven from one photobioreactor to another by the electric pump.

31. The plant according to any of the claims 19 to 30, wherein the photobioreactors comprise means for controlling the temperature and heat pumps of the type that are condensed by air, by water and/or are geothermal, in order to keep said parameter between 5 °C and 45 °C.

32. The plant according to any of the claims 19 to 31, which incorporates inline cleaning and disinfection means in the pipework and electric pumps in charge of the replacement and extraction of the culture of the photobioreactors as well as in the reject recovering processes and exchanges in the various tanks, said cleaning and disinfection means being selected from among the group formed by ultraviolet rays, active carbon filters and/or ozonation.

33. The plant according to any of the claims 19 to 32, further comprising a chamber for accumulating the stored and treated rejects, which is connected at the inlet thereof to the outlet of the two storage and treatment chambers of the first and second rejects, and at the outlet thereof to the means for injecting the water current into the photobioreactors.

34. The plant according to any of the claims 19 to 33, further comprising means for collecting leachates and spills formed by the means of leading and the means of pumping toward the means for injecting the sea, fresh and/or salty water current into the photobioreactor.

35. The plant according to the previous claim, wherein the means for collecting leachates and spills are connected to the accumulation chamber for the treated rejects, wherein they are stored along with the other rejects prior to being led to the means for injecting the water current into the photobioreactors.

36. The plant according to any of the claims 19 to 35, comprising process control means formed by sensors, flow meters, electrically operated valves and common elements of a SCADA system.

## Patentansprüche

1. Verfahren zur Herstellung von Biomasse und Produkten daraus, durch Kultivierung von einzelligen Algen in einem wässrigen Medium, welches mit einem CO₂-Strom in einer Reihe von Fotobioreaktoren gespeist wird, umfassend:
- Injizieren eines Stromes aus See-, Salz- oder frischem Wasser in die Fotobioreaktoren als ein Kulturmedium für die Algen, die zuvor mit Nährstoffen angereichert, mikrofiltriert und desinfiziert wurden,
- Injizieren wenigstens eines Stammes wenigstens einer Art von einzelligen Algen in die Fotobioreaktoren und Inkontaktbringen des Stammes mit dem angereicherten Wasserstrom unter Lichtbedingungen zum Wachstum der Kultur mittels Fotosynthese;
- Injizieren eines Gasstromes, enthaltend CO₂ in die Fotobioreaktoren,
**dadurch gekennzeichnet, dass** es des Weiteren umfasst
- Extrahieren eines Teiles der Algenkultur aus den Fotobioreaktoren, Lagern in einem Sammeltank und Senden zu einer separaten Einheit, um die Biomasse mittels mechanischer und/oder chemischer Trennung zu extrahieren, sodass die erhaltene Biomasse einen Feuchtigkeitsgrad zwischen 50 % und 90 % aufweist;
- Unterwerfen der nassen Biomasse einem Dekantieren und/oder einer Kavitation in einer getrennten Einheit, um einen Teil der Lipide und/oder Fettsäuren und andere Mehrwertprodukte zusammen mit einem ersten Ausschuss zu extrahieren, Leiten der übrig bleibenden Biomasse zu dem folgenden Schritt;
- Unterwerfen der übrig bleibenden Biomasse von dem vorausgehenden Schritt einer direkten thermischen Verflüssigung bei niedriger Temperatur in einer getrennten Einheit, um ein Biocrude und einen zweiten Ausschuss zu erzeugen, alles bei einer Temperatur zwischen 250 °C und 350 °C und einem Druck zwischen 150 und 210 bar, wobei beide Grenzen eingeschlossen sind, über einen Zeitraum zwischen 1 und 120 Minuten;
- Rückgewinnen des Ausschusses sowohl aus dem Schritt der Lipid-, Fettsäure- und anderer Mehrwertproduktextraktion und dem nachfolgenden Schritt der thermochemischen Behandlung unter subkritischen Bedingungen, Lagern jedes Ausschusses in separaten Kammern und Unterwerfen dieser einer physiochemischen Behandlung, basierend auf dem Einstellen des Salzgehaltes, pH-Wertes, Mikrofiltration, Desinfektion und Zugabe von Nährstoffen, zur nachfolgenden Wiedereinführung in die Kultur.

2. Verfahren nach dem vorangehenden Anspruch, wobei jeder der Ausschusse in einer getrennten Einheit unter kontinuierlichem Rühren und Belüftung oder mit einer Mischung aus Luft und CO₂ vor der Wiedereinführung gelagert wird.

3. Verfahren nach einem der vorangehenden Ansprüche, wobei der Gasstrom ein CO₂-Strom oder eine Mischung aus Gasen aus einem Verbrennungsverfahren ist.

4. Verfahren nach einem der vorangehenden Ansprüche, wobei der Gasstrom gekühlt, gewaschen, unter Druck gesetzt wird, und wobei das CO₂ von den anderen Gasen vor der Einführung dieser in die Fotobioreaktoren getrennt wird.

5. Verfahren nach einem der vorangehenden Ansprüche, wobei der Gasstrom vor der Einführung in die Fotobioreaktoren mit Druckluft gemischt wird.

6. Verfahren nach einem der vorangehenden Ansprüche, wobei der Gasstrom in einer radialen und diskontinuierlichen Weise in die Fotobioreaktoren injiziert wird, in Pulsen von zwischen 1 bis 3600 Sekunden in Zeitintervallen zwischen 1 und 3600 Sekunden, wobei beide Grenzen eingeschlossen sind.

7. Verfahren nach einem der vorangehenden Ansprüche, wobei der Gasstrom in einer Menge injiziert wird, umfassend zwischen 0,2 m³/m³ der Kultur und 2 m³/m³ der Kultur.

8. Verfahren nach einem der vorangehenden Ansprüche, wobei die Nährstoffe, mit denen der Wasserstrom angereichert wird, Makronährstoffe sind, Mikronährstoffe und/oder Spurenelemente, in einem Prozentanteil zwischen 10 % und 30 Gew.-% der erhaltenen Biomasse.

9. Verfahren nach dem vorangehenden Anspruch, wobei die Nährstoffe gewählt werden aus stickstoffhaltigen Salzen, wie Nitrat und Ammonium, Phosphaten und jeder Kombination dieser.

10. Verfahren nach einem der vorangehenden Ansprüche, wobei der Wasserstrom im Inneren der Fotobioreaktoren in der entgegengesetzten Richtung der Injektion des Gasstromes gedreht wird, wenn die zwei in Kontakt gebracht werden.

11. Verfahren nach einem der vorangehenden Ansprüche, wobei die Kultur in den Fotobioreaktoren auf einer Temperatur zwischen 5 °C und 45 °C gehalten wird.

12. Verfahren nach einem der vorangehenden Ansprüche, wobei die Algenkultur in den Fotobioreaktoren gehalten wird, bis eine Konzentration zwischen 100 Millionen Zellen/ml und 600 Millionen Zellen/ml erreicht wird.

13. Verfahren nach einem der vorangehenden Ansprüche, wobei täglich eine Kulturmenge zwischen 2 % und 50 % des gesamten Volumens im Inneren der Fotobioreaktoren aus den Fotobioreaktoren extrahiert wird.

14. Verfahren nach einem der vorangehenden Ansprüche, wobei die mechanische Trennung zur Erhaltung der Biomasse mittels eines Verfahrens durchgeführt wird, gewählt aus der Gruppe bestehend aus Zentrifugieren, Superzentrifugieren, Dekantieren und/oder Filtrieren, und die chemische Trennung durch Ausflocken durchgeführt wird.

15. Verfahren nach einem der vorangehenden Ansprüche, wobei ein Schaum, welcher auf der oberen Schicht des Wasserstromes im Inneren des Fotobioreaktors als eine Folge der Wiedereinführung des behandelten Ausschusses gebildet wird, gesammelt wird, wobei der Schaum Biomasse und/oder Nährstoffe in einem Prozentanteil zwischen 1 % und 5 % des in den Fotobioreaktoren existierenden Gesamtvolumens, umfasst.

16. Verfahren nach einem der vorangehenden Ansprüche, wobei der Algenstrom gewählt ist aus der Gruppe bestehend aus Cyanobacteria, Chlorophyceae, Rhodophyceae, Eustigmatophyceae, Diatome, Dinophyceae, Bacillariophyceae, Xanthophyten und Phaeophyceae und jeder Kombination dieser.

17. Verfahren nach einem der vorangehenden Ansprüche, wobei das Biocrude einer Kavitation, Dekantieren, Drücken und/oder thermischen Behandlung in einer separaten Einheit unterworfen wird, um einen Biokraftstoff und einen Ausschuss zu erhalten.

18. Verfahren nach dem vorangehenden Anspruch, wobei der Ausschuss, welcher zusammen mit dem Biokraftstoff erhalten wird, unter kontinuierlichem Rühren und unter Belüftung oder mit einer Mischung aus Luft und CO₂ in eine Speichereinheit geführt und einer physiochemischen Behandlung unterworfen wird, basierend auf dem Einstellen des Salzgehaltes, des pH-Wertes, Mikrofiltration, Desinfektion und Zugabe von Nährstoffen, zur nachfolgenden Wiedereinführung in die Kultur.

19. Anlage zur Herstellung von Biomasse und Produkten aus dieser, durch Kultivieren wenigstens einer einzelligen Algenart in einem wässrigen Medium, zugeführt mit einem Gasstrom, enthaltend CO₂, in einer Reihe von Fotobioreaktoren gemäß des in einem der vorangehenden Ansprüchen beschriebenen Verfahrens, umfassend:
- wenigstens eine Einheit zum Sammeln, Lagern und Injizieren von See-, frischem und/oder Salzwasserstrom, verbunden mit einer Nährstoffinjektionseinheit, welche Nährstoffe in den Strom injiziert, bevor dieser in die Fotobioreaktoren eintritt, und mit einer Mikrofiltrier- und Desinfiziereinheit;
- wenigstens eine Einheit zum Sammeln, Lagern und Injizieren des Gasstromes, enthaltend CO₂ in die Fotobioreaktoren;
wobei beide Einheiten verbunden sind mit
- einer Reihe von zwei oder mehr Fotobioreaktoren des ringförmigen vertikalen und kreisförmigen Typs, welche miteinander verbunden sind, um Biomasse herzustellen, durch Kultivieren wenigstens einer Art einer einzelligen Alge;
**dadurch gekennzeichnet, dass** sie des Weiteren umfasst
- Mittel zum Extrahieren eines Teiles der Kultur aus dem Inneren der Fotobioreaktoren und einen Sammeltank dafür, verbunden mit einer mechanischen Extrahiereinheit und/oder einer chemischen Extrahiereinheit für nasse Biomasse;
- eine Einheit zur Extraktion von Lipiden und/oder Fettsäuren und/oder anderen Mehrwertprodukten durch Kavitation, Dekantieren und/oder Auflösen der Biomasse, verbunden mit
- einer thermochemischen Behandlungseinheit zur Herstellung von Biocrude mittels einer direkten thermischen Verflüssigung bei niedriger Temperatur der überschüssigen Biomasse in einer Einheit zum Extrahieren von Lipiden und/oder Fettsäuren und/oder anderen Mehrwertprodukten,
- eine Lagerkammer für den ersten Ausschuss, erzeugt in der mechanischen Extraktionseinheit und/oder in der chemischen Extraktionseinheit, zur Rezirkulation des Ausschusses zu der Einheit zum Injizieren des Wasserstromes in die Fotobioreaktoren nach dem Konditionieren desselben im Inneren der Lagerkammer; und
- eine Lagerkammer für den zweiten Ausstoß, erzeugt in der Einheit zur Extraktion von Lipiden und/oder Fettsäuren und/oder Mehrwertprodukten und in der thermochemischen Behandlungseinheit mit Mitteln zur Rezirkulation des Ausschusses in die Einheit zum Injizieren des Wasserstromes in die Fotobioreaktoren nach dem Konditionieren derselben in der Lagerkammer.

20. Anlage nach dem vorangehenden Anspruch, wobei die Lagereinheiten des Permeats und des Ausschusses Rührmittel und Mittel zur Belüftung oder zur Einführung von Luft und CO₂ in das Innere umfassen.

21. Anlage nach einem der Ansprüche 19 oder 20, des Weiteren umfassend eine Druckluftinjektionseinheit, verbunden mit der Sammel-, Lager- und Injektionseinheit des Gasstromes, enthaltend CO₂ vor dem Eintritt in die Fotobioreaktoren.

22. Anlage nach einem der Ansprüche 19 bis 21, wobei jeder der Fotobioreaktoren des Weiteren eine elektrische Pumpe umfasst, wodurch das Wasser in einer zu der Richtung der Gasstrominjektion entgegengesetzten Richtung dreht, wenn beide in Kontakt mit dem Inneren des Fotobioreaktors gebracht werden.

23. Anlage nach einem der Ansprüche 19 bis 22, umfassend zwischen 2 und 5000 Fotobioreaktoren.

24. Anlage nach einem der Ansprüche 19 bis 22, wobei die Fotobioreaktoren aus einem Außenrohr und einem Innenrohr bestehen, welche einen Raum zwischen diesen einschließen, in welchem das Wasser und der Gasstrom injiziert werden, und in welchem die Algen kultiviert werden, wobei der Raum unterteilt wird, in längliche ungerade Bereiche von 1 bis 9, und die Spanne des Durchlasses in den Rohren kleiner als 5 cm ist.

25. Anlage nach einem der Ansprüche 19 bis 22, wobei die Reihe der Fotobioreaktoren in drei Bereiche konfiguriert ist, wie nachfolgend definiert:
- einen Bereich zum Mischen des Gasstromes, des Wasserstromes und eines Stammes der zu kultivierenden Algen, bestehend aus einer Gruppe von Fotobioreaktoren;
verbunden mit
- einem Bereich zum Wachsen der Algenkultur, bestehend aus einer zweiten Gruppe von Fotobioreaktoren; und
- einem Bereich zum Extrahieren der Kultur aus dem Inneren der Fotobioreaktoren, bestehend aus einer dritten Gruppe von Fotobioreaktoren.

26. Anlage nach einem der Ansprüche 24 oder 25, wobei die Fotobioreaktoren des Weiteren eine automatische Einrichtung zum Reinigen der Wände der Rohre umfasst, welche den Raum zwischen dem Innenrohr und dem Außenrohr begrenzen, wobei die Reinigungseinrichtung aus einem elastomeren Abstreifer besteht, dessen Ebene dem Profil des Raumes zwischen den Rohren entspricht und eine Dicke zwischen 2 und 5 cm aufweist, mit einer zentralen Durchtrittsöffnung, welche einen unteren frustokonischen Bereich und einen oberen zylindrischen Bereich aufweist, wobei die Durchtrittsöffnung die Gesamtform eines invertierten Trichters aufweist, und mit einer Kugel, hergestellt aus einem Kunststoffmaterial mit einem größeren Durchmesser als der Durchmesser des zylindrischen Bereichs der Durchtrittsöffnung, welche sich im Inneren des durch den unteren frustokonischen Bereich der Durchtrittsöffnung definierten Raumes bewegt.

27. Anlage nach einem der Ansprüche 19 bis 26, wobei die Fotobioreaktoren des Weiteren ein durchscheinendes konzentrisches Rohr umfassen, welches die Außenwand des Außenrohres bedeckt.

28. Anlage nach einem der Ansprüche 19 bis 27, wobei die Fotobioreaktoren des Weiteren Mittel umfassen, um einen Schaum zu sammeln, welcher auf der oberen Schicht des Wasserstroms gebildet wird, bestehend aus Rohrleitungen an der Oberseite des Fotobioreaktors und Mitteln zum Führen des Schaums in den Sammelbehälter der aus dem Fotobioreaktor extrahierten Kultur, die Rohre und elektrische Pumpen sind.

29. Anlage nach einem der Ansprüche 19 bis 28, wobei die Fotobioreaktoren in ihrem oberen Teil Mittel zum Sammeln des überschüssigen CO₂ umfassen, welches nicht von der Algenkultur verbraucht wurde, und Mittel um das überschüssige Gas in die Gaseinheit zu führen, um die Gase zu sammeln und in die Fotobioreaktoren zu injizieren.

30. Anlage nach einem der Ansprüche 19 bis 29, wobei die Fotobioreaktoren miteinander mittels Rohrleitungen verbunden sind, welche unter der Erde begrabener sind, in welcher die Anlage angeordnet ist, wobei der Wasserstrom mit der Kultur von einem Fotobioreaktor zu dem anderen mittels einer elektrischen Pumpe befördert wird.

31. Anlage nach einem der Ansprüche 19 bis 30, wobei die Fotobioreaktoren Mittel zur Steuerung der Temperatur und Wärmepumpen umfassen, welche von Luft, durch Wasser kondensiert werden und/oder geothermisch sind, um den Parameter zwischen 5 °C und 45 °C zu halten.

32. Anlage nach einem der Ansprüche 19 bis 31, umfassend das Inline-Reinigungs- und Desinfektionsmittel in den Rohren und elektrischen Pumpen, welche für den Ersatz und die Extraktion der Kultur der Fotobioreaktoren, wie auch die Ausschussrückgewinnungsverfahren und Austausche in den unterschiedlichen Behältern verantwortlich sind, wobei die Reinigungs- und Desinfektionsmittel gewählt werden aus der Gruppe, gebildet durch ultraviolette Strahlen, aktive Kohlenstofffilter und/oder Ozonierung.

33. Anlage nach einem der Ansprüche 19 bis 32, des Weiteren umfassend eine Kammer zum Sammeln des gelagerten und behandelten Ausschusses, verbunden an dem Einlass mit dem Auslass der zwei Lager- und Behandlungskammern des ersten und zweiten Ausschusses, und an dem Auslass mit Mitteln zum Injizieren des Wasserstroms in die Fotobioreaktoren.

34. Anlage nach einem der Ansprüche 19 bis 33, des Weiteren umfassend Mittel zum Sammeln von Sickerwasser und Leckagen, gebildet durch Mittel zum Führen und Mittel zum Pumpen in Richtung der Mittel zum Injizieren des See-, Frisch- und/oder Salzwasserstromes in die Fotobioreaktoren.

35. Anlage nach dem vorangehenden Anspruch, wobei die Mittel zum Sammeln des Sickerwassers und der Leckage mit der Sammelkammer für den behandelten Ausschuss verbunden sind, in welcher sie zusammen mit anderem Ausschuss gelagert werden, bevor sie zu den Mitteln zum Injizieren des Wasserstroms in die Fotobioreaktoren geführt werden.

36. Anlage nach einem der Ansprüche 19 bis 35, umfassend Verfahrenssteuermittel, gebildet durch Sensoren, Flussmeter, elektrisch betriebene Ventile und übliche Elemente eines SCADA-Systems.

## Revendications

1. Procédé de production de biomasse et de produits dérivés de celle-ci au moyen de culture d'algues unicellulaires dans un milieu aqueux alimenté avec un courant de CO₂ dans un jeu de photobioréacteurs, comprenant :
- l'injection d'un courant d'eau de mer, salée ou fraîche dans les photobioréacteurs comme un milieu de culture pour les algues, préalablement enrichie avec des nutriments, micro filtrée et désinfectée,
- l'injection d'une souche d'au moins une espèce d'algues unicellulaires dans les photobioréacteurs, et la mise en contact de ladite souche avec le courant d'eau enrichie dans des conditions de lumière pour la culture pour une croissance au moyen de photosynthèse ;
- l'injection d'un courant de gaz contenant CO₂ dans les photobioréacteurs,
**caractérisé en ce qu'**il comprend de plus
- l'extraction d'une partie de la culture d'algues des photobioréacteurs, le stockage dans un récipient d'accumulation et l'envoi vers une unité séparée pour extraire la biomasse au moyen de séparation mécanique et/ou chimique, de sorte que la biomasse obtenue présente un degré d'humidité compris entre 50 % et 90 % ;
- la soumission de la biomasse humide à une décantation et/ou une cavitation dans une unité séparée afin d'extraire une partie des lipides et/ou des acides gras et d'autres produits de valeur ajoutée avec un premier rejet, le passage de la biomasse restante vers l'étape suivante ;
- la soumission de la biomasse restante de l'étape précédente a une liquéfaction thermique, à faible température, directe dans une unité séparée afin de produire un produit biobrut et un second rejet, tout ceci à une température de 250°C à 350°C et une pression de 150 à 210 bars, les deux limites incluses, sur une durée de 1 à 120 minutes ;
- la récupération des rejets à la fois de l'étape d'extraction de lipides, d'acides gras et d'autres produits de valeur ajoutée et de l'étape subséquente de traitement thermochimique dans des conditions sous critiques, le stockage de chaque rejet dans des chambres séparées et la soumission à un traitement physicochimique basé sur l'ajustement de la salinité, du pH, une microfiltration, une désinfection et une addition de nutriments, pour une réintroduction subséquente dans la culture.

2. Procédé selon la revendication précédente, dans lequel chacun des rejets est stocké dans une unité séparée sous agitation et aération continues ou avec un mélange d'air et de CO₂ avant réincorporation.

3. Procédé selon l'une quelconque des revendications précédentes, dans lequel le courant de gaz est un courant de CO₂ ou un mélange de gaz provenant d'un procédé de combustion.

4. Procédé selon l'une quelconque des revendications précédentes, dans lequel le courant de gaz est refroidi, lavé, comprimé et le CO₂ est séparé des autres gaz avant son introduction dans les photobioréacteurs.

5. Procédé selon l'une quelconque des revendications précédentes, dans lequel le courant de gaz est mélangé avec de l'air comprimé avant son introduction dans les photobioréacteurs.

6. Procédé selon l'une quelconque des revendications précédentes, dans lequel le courant de gaz est injecté de manière radiale et discontinue dans le photobioréacteur, en pulsations durant de 1 à 3 600 secondes à des intervalles de temps de 1 à 3 600 secondes, les deux limites étant incluses.

7. Procédé selon l'une quelconque des revendications précédentes, dans lequel le courant de gaz est injecté dans une quantité de 0,2 m³/m³ de culture à 2 m³/m³ de culture.

8. Procédé selon l'une quelconque des revendications précédentes, dans lequel les nutriments avec lesquels le courant d'eau est enrichi sont des macronutriments, des micronutriments et/ou des éléments de traces, dans un pourcentage de 10 % à 30 % en masse de biomasse obtenue.

9. Procédé selon la revendication précédente, dans lequel les nutriments sont choisis parmi des sels azotés, tel que nitrate et ammonium, des phosphates et une combinaison quelconque de ceux-ci.

10. Procédé selon l'une quelconque des revendications précédentes, dans lequel le courant d'eau est mis en rotation à l'intérieur des photobioréacteurs dans la direction opposée de l'injection de courant de gaz lorsque les deux d'entre eux sont mis en contact.

11. Procédé selon l'une quelconque des revendications précédentes, dans lequel la culture est maintenue à l'intérieur des photobioréacteurs à une température de 5°C à 45°C.

12. Procédé selon l'une quelconque des revendications précédentes, dans lequel la culture d'algues est maintenue à l'intérieur des photobioréacteurs jusqu'à ce qu'une concentration de 100 millions de cellules/ml à 600 millions de cellules/ml est atteinte.

13. Procédé selon l'une quelconque des revendications précédentes, dans lequel une quantité de culture de 2 % à 50 % du volume total à l'intérieur des photobioréacteurs est extraite journalièrement des photobioréacteurs.

14. Procédé selon l'une quelconque des revendications précédentes, dans lequel la séparation mécanique pour obtenir la biomasse est réalisée au moyen d'un des procédés choisis dans le groupe constitué de centrifugation, super-centrifugation, décantation et/ou filtration, et la séparation chimique est réalisée au moyen de floculation.

15. Procédé selon l'une quelconque des revendications précédentes, dans lequel une mousse qui est formée sur la couche supérieure du courant d'eau à l'intérieur du photobioréacteur comme une conséquence de la réintroduction dans celui-ci du rejet traité, est recueillie, ladite mousse contenant de la biomasse et/ou des nutriments dans un pourcentage de 1 % à 5 % du volume total existant dans les photobioréacteurs.

16. Procédé selon l'une quelconque des revendications précédentes, dans lequel la souche d'algues est choisie dans le groupe constitué de Cyanobacteria, Chlorophyceae, Rhodophyceae, Eustigmatophyceae, des diatomes, Dinophyceae, Bacillariophyceae, Xanthophytes et Phaeophyceae, et d'une combinaison quelconque de ceux-ci.

17. Procédé selon l'une quelconque des revendications précédentes, le produit biobrut est soumis à une cavitation, une décantation, une compression et/ou un traitement thermique dans une unité séparée afin d'obtenir du biocombustible et un rejet.

18. Procédé selon la revendication précédente, dans lequel le rejet qui est obtenu avec le biocombustible est amené vers une unité de stockage sous agitation continue et avec aération ou avec un mélange d'air et de CO₂, et est soumis à un traitement physicochimique basé sur l'ajustement de la salinité, du pH, une microfiltration, une désinfection et une addition de nutriments, pour une réintroduction subséquente dans la culture.

19. Installation de production d'une biomasse et de produits dérivés de celle-ci par culture d'au moins une espèce d'algues cellulaires dans un milieu aqueux alimenté avec un courant de gaz contenant CO₂ dans un jeu de photobioréacteurs selon le procédé décrit dans l'une quelconque des revendications précédentes, comprenant :
- au moins une unité de recueil, de stockage et d'injection du courant d'eau de mer, fraiche et/ou salée connectée à une unité d'injection de nutriments qui injecte des nutriments dans ledit courant avant l'entrée dans les photobioréacteurs, et à une unité de microfiltration et de désinfection ;
- au moins une unité de recueil, de stockage et d'injection du courant de gaz contenant CO₂ dans les photobioréacteurs ;
les deux unités étant connectées à
- un jeu de deux ou plusieurs photobioréacteurs du type annulaire vertical et circulaire connectés les uns aux autres pour produire une biomasse au moyen de culture d'au moins une espèce d'algues unicellulaires ;
**caractérisée en ce qu'**elle comprend de plus
- un moyen d'extraction d'une partie de la culture de l'intérieur des photobioréacteurs et d'un récipient d'accumulation de celle-ci, connecté à une unité d'extraction mécanique et/ou à une unité d'extraction chimique de biomasse humide ;
- une unité d'extraction de lipides et/ou d'acides gras et/ou d'autres produits de valeur ajoutée au moyen de cavitation, décantation et/ou dissolution de la biomasse, connectée à
- une unité de traitement thermochimique pour produire un produit biobrut au moyen de liquéfaction thermique, à faible température, directe de la biomasse en surplus dans l'unité d'extraction de lipides et/ou d'acides gras et/ou d'autres produits de valeur ajoutée,
- une chambre de stockage pour le premier rejet produit dans l'unité d'extraction mécanique et/ou dans la chambre d'extraction chimique, avec un moyen de recirculation dudit rejet vers l'unité d'injection du courant d'eau dans les photobioréacteurs après conditionnement de celui-ci à l'intérieur de la chambre de stockage ; et
- une chambre de stockage pour le second rejet produit dans l'unité d'extraction de lipides et/ou d'acides gras et/ou de produits de valeur ajoutée et dans l'unité de traitement thermochimique, avec un moyen de recirculation dudit rejet vers l'unité d'injection du courant d'eau dans les photobioréacteurs après conditionnement de celui-ci à l'intérieur de la chambre de stockage.

20. Installation selon la revendication précédente, dans laquelle les unités de stockage du perméat et du rejet comprennent un moyen d'agitation et un moyen d'aération ou d'injection d'air et de CO₂ dans celles-ci.

21. Installation selon l'une quelconque des revendications 19 ou 20, laquelle comprend de plus une unité d'injection d'air comprimé connectée à l'unité de recueil, de stockage et d'injection du courant de gaz contenant CO₂ avant l'entrée dans les photobioréacteurs.

22. Installation selon l'une quelconque des revendications 19 à 21, dans laquelle chaque photobioréacteur comprend de plus une pompe électrique qui met l'eau en rotation dans la direction opposée de celle de l'injection de courant de gaz lorsque les deux sont mis en contact à l'intérieur des photobioréacteurs.

23. Installation selon l'une quelconque des revendications 19 à 22, comprenant de 2 à 5 000 photobioréacteurs.

24. Installation selon l'une quelconque des revendications 19 à 22, dans laquelle les photobioréacteurs sont constitués d'un tube externe et d'un tube interne délimitant un espace entre ceux-ci dans lequel les courants d'eau et de gaz sont injectés et où les algues sont cultivées, ledit espace étant divisé en un nombre impair de sections longitudinales de 1 à 9, et la travée du passage à l'intérieur des tubes étant inférieur à 5 cm.

25. Installation selon l'une quelconque des revendications 19 à 22, dans lequel le jeu de photobioréacteurs est configuré en 3 zones définies comme suit :
- une zone de mélange du courant de gaz, du courant d'eau et d'une souche des algues à cultiver, laquelle est constituée par un groupe de photobioréacteurs ; connectée à
- une zone de croissance de la culture d'algues, qui est constituée d'un second groupe de photobioréacteurs ; et
- une zone d'extraction de la culture à partir de l'intérieur des photobioréacteurs, qui est constituée d'un troisième groupe de photobioréacteurs.

26. Installation selon l'une quelconque des revendications 24 à 25, dans laquelle les photobioréacteurs comprennent de plus un dispositif automatisé pour nettoyer les parois des tubes délimitant l'espace entre le tube interne et le tube externe, ledit dispositif de nettoyage étant constitué d'un dispositif de grattage élastomère dont le plan correspond au profil de l'espace entre les tubes et à une épaisseur de 2 à 5 cm, avec un trou de passage central qui présente une section tronconique inférieure et une section cylindrique supérieure, le trou présentant la forme globale d'un entonnoir inversé ; et d'une sphère constituée de matière plastique avec un diamètre supérieur au diamètre de la section cylindrique du trou, laquelle se déplace à l'intérieur de l'espace défini par la section tronconique inférieure du trou de passage.

27. Installation selon l'une quelconque des revendications 19 à 26, dans laquelle les photobioréacteurs comprennent de plus un tube concentrique transparent recouvrant la paroi externe du tube externe.

28. Installation selon l'une quelconque des revendications 19 à 27, dans laquelle les photobioréacteurs comprennent de plus un moyen de recueil de mousse qui est formée sur la couche supérieure du courant d'eau, constitué d'une tuyauterie en haut du photobioréacteur, et un moyen d'amenée de ladite mousse vers le récipient d'accumulation pour la culture extraite du photobioréacteur, lesquels sont des tuyaux et des pompes électriques.

29. Installation selon l'une quelconque des revendications 19 à 28, dans laquelle les photobioréacteurs comprennent dans leur partie supérieure un moyen de recueil de CO₂ en surplus non consommé par la culture d'algues et un moyen d'amenée dudit gaz en surplus vers l'unité de gaz pour recueillir et injecter des gaz dans les photobioréacteurs.

30. Installation selon l'une quelconque des revendications 19 à 29, dans laquelle les photobioréacteurs sont connectés les uns aux autres au moyen de tuyaux enterrés sous le sol où l'installation est située, le courant d'eau avec la culture étant conduit d'un photobioréacteur à l'autre par la pompe électrique.

31. Installation selon l'une quelconque des revendications 19 à 30, dans laquelle les photobioréacteurs comprennent un moyen de contrôle de la température et des pompes à chaleur du type tel qu'elles sont condensées par l'air, par l'eau et/ou sont géothermiques, afin de maintenir ledit paramètre de 5°C à 45°C.

32. Installation selon l'une quelconque des revendications 19 à 31, laquelle incorpore un moyen de nettoyage et de désinfection en ligne dans la tuyauterie et les pompes électriques en charge du remplacement et de l'extraction de la culture des photobioréacteurs ainsi que dans les procédés de récupération de rejet et les échanges dans les différents récipients, ledit moyen de nettoyage et de désinfection étant choisi dans le groupe formé par des rayons ultra-violets, des filtres au charbon actif et/ou une ozonisation.

33. Installation selon l'une quelconque des revendications 19 à 32, comprenant de plus une chambre d'accumulation des rejets stockés et traités, laquelle est connectée à l'entrée de celle-ci à la sortie des deux chambres de stockage et de traitement des premier et second rejets, et à la sortie de celle-ci au moyen d'injection du courant d'eau dans les photobioréacteurs.

34. Installation selon l'une quelconque des revendications 19 à 33, comprenant de plus un moyen de recueil de lixiviats et de déversements formés par le moyen d'amenée et le moyen de pompage vers le moyen d'injection du courant d'eau de mer, fraîche et/ou salée dans le photobioréacteur.

35. Installation selon la revendication précédente, dans laquelle le moyen de recueil de lixiviats et de déversements est connecté à la chambre d'accumulation pour les rejets traités, dans laquelle ils sont stockés avec les autres rejets avant d'être amenés vers le moyen d'injection du courant d'eau dans les photobioréacteurs.

36. Installation selon l'une quelconque des revendications 19 à 35, comprenant un moyen de contrôle de procédé formé par des capteurs, des débitmètres, des vannes fonctionnant électriquement et des éléments classiques d'un système SCADA.
